(19) Europäisches Patentamt
European Patent Office
Office européen des brevets

(11) EP 2 921 181 A1

(12) **EUROPEAN PATENT APPLICATION**

(43) Date of publication:
**23.09.2015 Bulletin 2015/39**

(51) Int Cl.:
***A61K 51/00*** *(2006.01)* ***A61K 31/198*** *(2006.01)*

(21) Application number: **14193736.7**

(22) Date of filing: **30.06.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.06.2005 US 695503 P**

(62) Document number(s) of the earlier application(s) in accordance with Art. 76 EPC:
**13194043.9**
**06780854.3 / 1 898 961**

(71) Applicant: **Otsuka America Pharmaceutical, Inc.**
**Rockville, MD 20850 (US)**

(72) Inventors:
- **Modak, Anil**
  **Haverhill, MA Massachusetts 01830 (US)**
- **Kurogi, Yasuhisa**
  **Princeton, NJ New Jersey 08540 (US)**

(74) Representative: **inCompass IP Europe Limited**
**4 Bloomsbury Place**
**London WC1A 2QA (GB)**

Remarks:
This application was filed on 18-11-2014 as a divisional application to the application mentioned under INID code 62.

# (54) Method to individualize levodopa/carbidopa therapy using a breath test

(57) A preparation for determining levodopa metabolic capacity, comprising as an active ingredient a levodopa in which at least one of the carbon or oxygen atoms is labeled with an isotope, wherein the preparation is capable of producing isotope-labeled $CO_2$ after administration to a mammalian subject. A method for determining levodopa metabolic capacity in a mammalian subject, comprising the steps of administering a preparation according to claim 1 to a mammalian subject, and measuring the excretion behavior of an isotope-labeled metabolite excreted from the body. A method for determining the efficacy of a dopamine decarboxylase inhibitor to treat a medical condition in a first mammalian subject, the method comprising (a) administering to the first subject the dopamine decarboxylase inhibitor and a levodopa in which at least one of the carbon or oxygen atoms is labeled with an isotope, wherein the levodopa is capable of producing isotope labeled $CO_2$; (b) determining the level of levodopa metabolic capacity by measuring isotope labeled $CO_2$ produced in the first subject; and (c) comparing the level of levodopa metabolic capacity of the first subject to level of a reference standard levodopa metabolic capacity, wherein a similarity in the level of levodopa metabolic capacity of the first subject compared to level of a reference standard levodopa metabolic capacity indicates that the dopamine decarboxylase inhibitor is effective to treat the medical condition in the first mammalian subject.

FIGURE 1

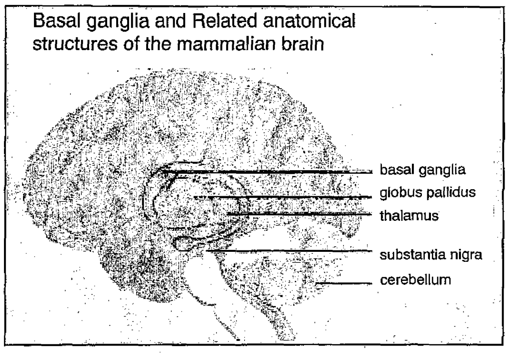


EP 2 921 181 A1

## Description

The present invention relates, generally to a method of determining and assessing L-3,4-dihydroxyphenylalanine (*a.k.a.*, Levodopa; L-dopa; or LD) metabolic capacity or dopamine decarboxylase (DDC) activity in an individual mammalian subject via a breath assay, by determining the relative amount of $^{13}CO_2$ exhaled by the subject upon intravenous or oral administration of a $^{13}C$-labeled substrate, such as levodopa. The present invention is useful as an *in vivo* phenotype assay for optimizing the dose and timing of administration of dopamine decarboxylase (DDC) inhibitor, such as Carbidopa (CD) for systemic suppression of dopamine metabolism in Parkinson's disease patients, by evaluating DDC enzyme activity using the metabolite $^{13}CO_2$ in expired breath.

## BACKGROUND

Conventional medical approaches to diagnosis and treatment of disease is based on clinical data alone, or made in conjunction with a diagnostic test(s). Such traditional practices often lead to therapeutic choices that are not optimal for the efficacy of the prescribed drug therapy or to minimize the likelihood of side effects for an individual subject. Therapy specific diagnostics (a.k.a., theranostics) is an emerging medical technology field, which provides tests useful to diagnose a disease, choose the correct treatment regime and monitor a subject's response. That is, theranostics are useful to predict and assess drug response in individual subjects, i.e., individualized medicine. For example, knowledge of a patient's phenotype or the drug metabolizing capacity can enable physicians to individualize therapy thereby avoiding potential drug related toxicity in poor metabolizers and increasing efficacy. Theranostic tests are also useful to select subjects for treatments that are particularly likely to benefit from the treatment or to provide an early and objective indication of treatment efficacy in individual subjects, so that the treatment can be altered with a minimum of delay. Theranostic tests may be developed in any suitable diagnostic testing format, which include, but is not limited to, e.g., non-invasive breath tests, immunohistochemical tests, clinical chemistry, immunoassay, cell-based technologies, and nucleic acid tests.

One conventional medical treatment in need of a reliable theranostic test is the therapeutic treatment of Parkinsons disease (hereinafter sometimes referred to as "PD") with a combination of levodopa (hereinafter sometimes referred to as "LD") and carbidopa (hereinafter sometimes referred to as "CD") (Deleu et al., Eur J Clin Pharmacol. 41: 453-8, 1991).

The symptoms of PD result largely from the loss of dopamine-producing cells in the substantia nigra region of the mammalian brain. Since dopamine does not cross the blood brain barrier (BBB), the use of LD as a means of neurotransmitter replacement therapy in PD is now a standard clinical regimen. When LD is taken orally, some of the drug crosses the BBB into the central nervous system and is enzymatically converted to dopamine. However, LD is decarboxylated systemically in liver, kidney, the gastrointestinal tract and endothelial cells of capillary walls. This peripheral decarboxylation is responsible for significant side effects in subjects that include nausea, vomiting, cardiac arrhythmias and hypotension. In addition, any LD that is converted to dopamine systemically cannot enter the brain, resulting in diminished central nervous system dopamine level. CD is an inhibitor of aromatic amino acid decarboxylation that is useful to inhibit peripheral LD decarboxylation by DDC. Unlike LD, CD does not cross the blood-brain barrier. CD is routinely administered as a second drug with LD to inhibit peripheral decarboxylation in the treatment of PD. That is, CD prevents the breakdown of LD before it crosses into the brain.

The amount of LD entering a subject's brain is critical to optimal control of LD therapeutic levels and the consequent clinical benefit of LD. Effective administration of CD to inhibit peripheral decarboxylation of LD is an important factor affecting the amount of LD entering the brain of a subject. For example, where less CD is available or its inhibitory action compromised, peripheral metabolism of LD by dopamine decarboxylase (hereinafter sometimes referred to as "DDC") is greater and thus less LD is available for DDC-mediated enzymatic conversion in the CNS. Determination of optimal CD dosage for refinement of LD therapeutic delivery is confounded by individual subject variability in CD absorption and/or responsiveness. Studies employing stable isotope-labeled LD (Durso et al., Clin. Pharmacol., 40: 854-860, 2000) showed that absorption of CD is variable among human subjects with significant consequence to the degree of peripheral decarboxylation inhibition among subjects as well as the subsequent level of dopamine replacement in brain. Subjects can be classified as "good/rapid" CD absorbers or "poor/slow" CD absorbers (Durso et al., J. Clin, Pharmacol., 40: 854-860, 2000). The level of DDC inhibition of individual subjects to the same dose of CD also varies. *A priori* there is no way of knowing whether a subject is "CD sensitive" and will respond well to CD administration or "CD insensitive" and not show marked inhibition of peripheral DDC with CD administration.

A substantial clinical concern regarding LD is its association with the development of motor complications after long-term use in subjects suffering from PD. Pulsatile dopaminergic stimulation as a result of erratic absorption and the short half-life of LD have been central issues in attempts to explain this occurrence. Evidence suggests that altering the delivery of LD to provide a more continuous supply of this drug to the brain may result in improved control of PD symptoms. Accordingly, there is a need in the art to develop new diagnostic assays useful to assess the dose dependence of CD

on peripheral LD decarboxylation and LD uptake in the brain of individual subjects afflicted with PD in order to determine individual optimized LD and CD dosages.

GENERAL

The present invention relates generally to a diagnostic, noninvasive, in vivo phenotype test to evaluate DDC enzyme activity using enzyme substrate labeled with isotope incorporated at least at one specific position. The present invention utilizes the DDC enzyme-substrate interaction such that there is release of stable isotope labeled $CO_2$ in the expired breath of a mammalian subject.

In one aspect, the invention provides a preparation for determining LD metabolic capacity or DDC enzyme activity in a mammalian subject, comprising as an active ingredient an LD in which at least one of the carbon or oxygen atoms is labeled with an isotope, wherein the preparation is capable of producing isotope-labeled $CO_2$ after administration to a mammalian subject. In one embodiment, the preparation is labeled with at least one isotope selected from $^{13}C$; $^{14}C$; and $^{18}O$.

In another aspect, the invention provides a method for determining LD metabolic capacity, comprising administering a preparation comprising as an active ingredient an LD in which at least one of the carbon or oxygen atoms is labeled with an isotope, wherein the preparation is capable of producing isotope-labeled $CO_2$ after administration to a mammalian subject, and measuring the excretion behavior of an isotope-labeled metabolite excreted from the body. In one embodiment of the method, the isotope-labeled metabolite is excreted from the body as isotope-labeled $CO_2$ in the expired air.

In one embodiment, the method of the invention is a method for determining LD metabolic capacity in a mammalian subject, comprising administering to a mammalian subject a preparation comprising as an active ingredient an LD in which at least one of the carbon or oxygen atoms is labeled with an isotope, wherein the preparation is capable of producing isotope-labeled $CO_2$ after administration to a mammalian subject, measuring the excretion behavior of an isotope-labeled metabolite excreted from the body; and assessing the obtained excretion behavior in the subject. In one embodiment of-the method, a mammalian subject is administered a preparation comprising as an active ingredient an LD in which at least one of the carbon or oxygen atoms is labeled with an isotope, wherein the preparation is capable of producing isotope-labeled $CO_2$ after administration to a mammalian subject, the excretion behavior of isotope-labeled $CO_2$ in the expired air is measured, and the obtained excretion behavior of $CO_2$ in the subject is assessed. In one embodiment of the method, a mammalian subject is administered a preparation comprising as an active ingredient an LD in which at least one of the carbon or oxygen atoms is labeled with an isotope, wherein the preparation is capable of producing isotope-labeled $CO_2$ after administration to a mammalian subject, the excretion behavior of an isotope-labeled metabolite is measured, and the obtained excretion behavior in the subject or a pharmacokinetic parameter obtained therefrom is compared with the corresponding excretion behavior or parameter in a healthy subject with a normal LD metabolic capacity.

In one embodiment of the method, a mammalian subject is co-administered a DDC inhibitor CD along with a preparation comprising as an active ingredient an LD in which at least one of the carbon or oxygen atoms is labeled with an isotope, wherein the preparation is capable of producing isotope-labeled $CO_2$ after administration to a mammalian subject, the excretion behavior of an isotope-labeled metabolite is measured to determine the optimal dose of CD for suppressing DCC in the subject.

In one embodiment, the method of the invention is a method for determining the existence, nonexistence, or degree of LD metabolic disorder in a mammalian subject, comprising the steps of administering to the subject, a preparation comprising as an active ingredient an LD in which at least one of the carbon or oxygen atoms is labeled with an isotope, wherein the preparation is capable of producing isotope-labeled $CO_2$ after administration to a mammalian subject, measuring the excretion behavior of an isotope-labeled metabolite excreted from the body, and assessing the obtained excretion behavior in the subject.

In one embodiment, the method of the invention is a method for determining LD metabolic capacity, comprising administering to a mammalian subject a preparation comprising as an active ingredient an LD in which at least one of the carbon or oxygen atoms is labeled with an isotope, wherein the preparation is capable of producing isotope-labeled $CO_2$ after administration to the mammalian subject, and measuring the excretion behavior of an isotope-labeled metabolite excreted from the body. In one embodiment of the method, the isotope-labeled metabolite is excreted from the body as isotope-labeled $CO_2$ in the expired air.

In one embodiment, the method of the invention is a method for determining the efficacy of a DDC inhibitor to treat a medical condition in a first mammalian subject, comprising the steps of: (a) administering to the first subject the DDC inhibitor and an LD in which at least one of the carbon or oxygen atoms is labeled with an isotope, wherein the LD is capable of producing isotope labeled $CO_2$; (b) determining the level of LD metabolic capacity comprising the steps of measuring isotope labeled $CO_2$ produced in the first subject; and (c) comparing the level of LD metabolic capacity of the first subject to the level of a reference standard LD metabolic capacity, wherein a similarity in the level of LD metabolic

capacity of the first subject compared to level of LD metabolic capacity of the reference standard indicates that the DDC inhibitor is effective to treat the medical condition in the first mammalian subject.

In one embodiment, the method of the invention is a method for determining the optimal dose a DDC inhibitor to treat a medical condition in a first mammalian subject, comprising the steps of: (a) administering to the first subject the DDC inhibitor CD and an LD in which at least one of the carbon or oxygen atoms is labeled with an isotope, wherein the LD is capable of producing isotope labeled $CO_2$; (b) determining the level of LD metabolic capacity, an excretion behavior or a pharmacokinetic parameter by measuring isotope labeled $CO_2$ produced in the subject; and (c) determining the optimal dose of CD to maximize the efficacy of LD base on the obtained results in the step (b). The optimal dose of CD when used with LD will be effectively used to treat the medical condition in the mammalian subject.

In one embodiment of the method, the level of the reference standard LD metabolic capacity is the level of LD metabolic capacity of the first subject before administration of the DDC inhibitor. In one embodiment of the method, the level of the reference standard LD metabolic capacity is the average of the level of the LD metabolic capacity of a one or more second mammalian subject.

In one embodiment, the method of the invention is a method for selecting a prophylactic or therapeutic treatment for a subject, comprising: (a) determining the phenotype, including DDC enzyme activity or LD metabolic capacity, of the subject; (b) assigning the subject to a subject class based on the phenotype of the subject; and (c) selecting a prophylactic or therapeutic treatment based on the subject class, wherein the subject class comprises two or more individuals who display a level of DDC inhibition that is at least about 10 percent lower than a reference standard level of DDC inhibition. In one embodiment of the method, comprising the following step (c) in place of the above (c); step (c) selecting a prophylactic or therapeutic treatment based on the subject class, wherein the subject class comprises two or more individuals who display a level of DDC inhibition that is at least about 10% higher than a reference standard level of DDC inhibition. In one embodiment of the method, comprising the following step (c") in place of the above (c); step (c") selecting a prophylactic or therapeutic treatment based on the subject class, wherein the subject class comprises two or more individuals who display a level of DDC inhibition within at least about 10 percent of a reference standard level of DDC inhibition. In one embodiment of the method, the treatment is selected from administering a drug, selecting a drug to be administered selecting a drug dosage, and selecting the timing of a drug administration.

In one embodiment, the method of the invention is a method for selecting a prophylactic or therapeutic treatment for a subject, comprising the steps of: (a) administering to a subject the DDC inhibitor CD and an LD in which at least one of the carbon or oxygen atoms is labeled with an isotope, wherein the LD is capable of producing isotope labeled $CO_2$; (b) determining the level of LD metabolic inhibition by CD, by measuring isotope labeled $CO_2$ produced in the subject; and (c) selecting a prophylactic or therapeutic treatment, including the timing of a CD administration, based on the level of LD metabolic inhibition by CD in the subject.

In one embodiment, the method of the invention is a method for selecting a prophylactic or therapeutic treatment for a subject, comprising: (a) determining the phenotype, including DDC enzyme activity or LD metabolic activity of the subject; (b) assigning the subject to a subject class based on the phenotype of the subject; and (c) selecting a prophylactic or therapeutic treatment based on the subject class, wherein the subject class comprises two or more individuals who metabolize LD at a rate at least about 10 percent higher than a reference standard rate of LD metabolism. In one embodiment of the method, comprising the following step (c) in place of the above (c); step (c) selecting a prophylactic or therapeutic treatment based on the subject class, wherein the subject class comprises two or more individuals who metabolize LD at a rate at least about 10 percent lower than a reference standard rate of LD metabolism. In one embodiment of the method, comprising the following step (c") in place of the above (c); step (c") selecting a prophylactic or therapeutic treatment based on the subject class, wherein the subject class comprises two or more individuals who metabolize LD at a rate within at least about 10 percent of a reference standard rate of LD metabolism. In one embodiment of the method, the treatment is selected from administering a drug, selecting a drug to be administered, selecting a drug dosage, and selecting the timing of a drug administration.

In one embodiment of the method, the treatment is selected from administering a drug, selecting a drug to be administered, selecting a drug dosage, and selecting the timing of a drug administration.

In one embodiment, the method of the invention is a method for evaluating LD metabolic capacity, comprising the steps of: administering a $^{13}$C-labeled DDC substrate to a mammalian subject; measuring $^{13}CO_2$ exhaled by the subject; and determining LD metabolic capacity from the measured $^{13}CO_2$. In one embodiment of the method, the $^{13}$C-labeled DDC substrate is a $^{13}$C-labeled LD. In one embodiment of the method, the $^{13}$C-labeled DDC substrate is administered non-invasively. In one embodiment of the method, the $^{13}$C-labeled DDC substrate is administered intravenously or orally. In one embodiment of the method, the exhaled $^{13}CO_2$ is measured spectroscopically. In one embodiment of the method, the exhaled $^{13}CO_2$ is measured by infrared spectroscopy. In one embodiment of the method, the exhaled $^{13}CO_2$ is measured with a mass analyzer. In one embodiment of the method, the exhaled $^{13}CO_2$ is measured over at least three time periods to generate a dose response curve, and the LD metabolic capacity is determined from the area under the curve. In one embodiment of the method, the exhaled $^{13}CO_2$ is measured over at least two different dosages of the $^{13}$C-labeled DDC substrate. In one embodiment of the method, the exhaled $^{13}CO_2$ is measured during at least

the following time points: to, a time prior to ingesting the $^{13}C$-labeled substrate; $t_1$, a time after the $^{13}C$-labeled DDC substrate has been absorbed in the bloodstream of the subject; and $t_2$, a time during the first elimination phase. In one embodiment the method, the LD metabolic capacity is determined from as the a slope of $\delta^{13}CO_2$ at time points $t_1$ and $t_2$ calculated according to the following equation: slope = $[(\delta^{13}CO_2)_2-(\delta^{13}C0_2)_1]/(t_2-t_1)$- wherein $\delta^{13}CO_2$ is the amount of exhaled $^{13}CO_2$. In one embodiment of the method, an LD antagonist is administered to the subject before administrating a $^{13}C$-labeled DDC substrate.

In another aspect, the invention provides a kit comprising: a $^{13}C$-labeled DDC substrate; and instructions provided with the substrate that describes how to determine $^{13}C$-labeled LD metabolic capacity in a subject. In one embodiment of the kit, the $^{13}C$-labeled DDC substrate is $^{13}C$-labeled LD. In one embodiment of the kit, the kit further comprises at least three breath collection bags.

In one embodiment, the kit of the invention is a kit comprising: a $^{13}C$-labeled DDC substrate and at least one least one DDC inhibitor; and instructions provided with the substrate that describe how to determine antagonist dose and timing of inhibitor dose in a subject. In one embodiment of the kit, the $^{13}C$-labeled DDC substrate is $^{13}C$-labeled LD and the inhibitor is CD. In one embodiment of the kit, the kit further comprises at least six breath collection bags.

## BRIEF DESCRIPTION OF THE DRAWINGS

The drawing figures depict preferred embodiments by way of example, not by way of limitations. In the figures, like reference numerals refer to the same or similar elements.

Figure 1 is a schematic drawing of the basal ganglia and related anatomical structures of the mammalian brain.

Figure 2 is a schematic drawing of the metabolism of levodopa.

Figure 3 is a schematic drawing of the clinical effect achieved with levodopa therapy in patients with moderate Parkinson's disease (PD). It is a citation from Obeso JA et al. (Olanow CW, Obeso JA eds. Beyond the Decade of the Brain. Vol.2. Dopamine agonists in early Parkinson's disease. Kent. UK. Wells Medical Ltd. 1997. 11-35)

Figure 4 is a graph illustrating variable carbidopa (CD) absorption in human subjects expressed as a time course of serum carbidopa (CD) concentration (ng/ml) following 50 mg oral CD administration. Durso et al. (J. Clin. Pharmacol., 40: 854-860, 2000) showed a wide variation in CD absorption. Human subjects classified as "good/rapid" CD absorbers (N=4) are designated by a solid circles. Human subjects classified as "poor/slow" CD absorbers (N=5) are designated by open circles.

Figures 5 to 8 show graphs illustrating CD-mediated suppression of peripheral LD metabolism by DDC enzyme in human subjects. Figure 5 is a graph of the presence of $^{13}CO_2$ in expired breath samples expressed as delta over baseline (DOB) of a human subject (Vlt 1) pretreated with the indicated dosage of CD as a function of time (min). Figure 6 is a graph of the percentage dose recovery (PDR) of $^{13}C$- labeled LD as $^{13}CO_2$ in expired breath samples observed in a human subject (Vlt 1) pretreated with the indicated dosage of CD as a function of time (min). Figure 7 is a graph of the presence of $^{13}CO_2$ in expired breath samples expressed as DOB of a human subject (Vlt 2) pretreated with the indicated dosage of CD as a function of time (min). Figure 8 is a graph of PDR of $^{13}C$-labeled LD as $^{13}CO_2$ in expired breath samples observed in a human subject (Vlt 2) pretreated with the indicated dosage of CD as a function of time (min).

Figures 9 to 14 show graphs illustrating the effect of timing of carbidopa (CD) dosing on peripheral LD metabolism by DDC enzyme in human subjects. Figure 9 is a graph of the time course (min) of the appearance of $^{13}CO_2$ in breath samples expressed as DOB of a human subject (Vlt 1) receiving the indicated dosage of CD either 1 h prior to LD administration or simultaneously with this LD treatment. Figure 10 is a graph of the time course (min) of the PDR of $^{13}C$-labeled LD as $^{13}CO_2$ in breath samples in a human subject (Vlt 1) administered the indicated dosage of CD either 1 h prior to LD treatment or simultaneously with LD treatment. Figure 11 is a graph of the time course (min) of the appearance of $^{13}CO_2$ in breath samples expressed as DOB of a human subject (Vlt 2) receiving the indicated dosage of CD either 1 h prior to LD administration or simultaneously with LD treatment. Figure 12 is a graph of the time course (min) of the PDR of $^{13}C$-labeled LD as $^{13}CO_2$ in breath samples in a human subject (Vlt 2) administered the indicated dosage of CD either 1 h prior to LD treatment or simultaneously LD treatment. Figure 13 is a graph of the time course (min) of the appearance of $^{13}CO_2$ in breath samples expressed as DOB of a human subject (Vlt 2) receiving the indicated dosage of CD either 1 h prior to LD administration, 2 h prior to LD administration or simultaneously with LD treatment. Figure 14 is a graph of the time course (min) of the PDR of $^{13}C$-labeled LD as $^{13}CO_2$ in breath samples in a human subject (Vlt 2) administered the indicated dosage of CD either 1 h prior to LD

treatment, 2 h prior to LD treatment, or simultaneously with LD treatment.

DETAILED DESCRIPTION

It is to be appreciated that certain aspects, modes, embodiments, variations and features of the invention are described below in various levels of detail in order to provide a substantial understanding of the present invention. The present invention relates to a diagnostic, noninvasive, *in vivo* phenotype test to evaluate dopamine decarboxylase enzyme (DDC; EC 4.1.1.28) activity, using its enzyme substrate (e.g., levodopa, a.k.a., L-dopa or LD) labeled with isotope incorporated at least at one specific position. The present invention utilizes the DDC enzyme-substrate interaction such that there is release of stable isotope labeled $CO_2$ (e.g., $^{13}CO_2$) in the expired breath of a mammalian subject. The subsequent quantification of stable isotope labeled $CO_2$ allows for the indirect determination of pharmacokinetics of the substrate and the evaluation of DDC enzyme activity (i.e. LD metabolic capacity). In one embodiment, the invention provides a breath test for evaluation of peripheral DDC activity based on the oral or iv administration of a stable isotope $^{13}C$-labeled LD in combination with or without the DDC inhibitor carbidopa (CD; a.k.a., C-dopa), and measurement of the $^{13}CO_2/^{12}CO_2$ ratio in expired breath using commercially available instrumentation, e.g., mass or infrared (IR) spectrometers.

A substantial clinical concern regarding LD is its association with the development of motor complications after long-term use in neurotransmitter replacement therapy in subjects suffering from PD. Pulsatile dopaminergic stimulation as a result of erratic absorption and the short half-life of LD have been central issues in attempts to explain this occurrence. Evidence suggests that altering the delivery of LD to provide a more continuous supply of this drug to the brain may result in improved control of PD symptoms. The method of the invention solves a need in the art for a noninvasive method useful to define therapeutic regimens to modulate DDC activity (i.e. LD metabolic capacity) in a subject that yield more controlled and continuous LD to the brain of the subject. For example, in one embodiment, the method of the invention is useful to determine the CD dose to completely suppress peripheral DDC activity in a subject. In another embodiment, the method of the invention is also useful to determine the ideal timing of CD administration before LD dose.

The diagnostic test (breath test) of the present invention is advantageous as it is rapid and noninvasive, therefore placing less burden on the subject to give an accurate in vivo assessment of DDC enzyme activity both safely and without side effects. Accordingly, the various aspects of the present invention relate to preparations, diagnostic/theranostic methods and kits useful to identify individuals predisposed to disease or to classify individuals with regard to drug responsiveness, side effects, or optimal drug dose. Various particular embodiments that illustrate these aspects follow.

I. Definitions

As used herein, the term "clinical response" means any or all of the following: a quantitative measure of the response, no response, and adverse response (i.e., side effects).

As used herein, the term "effective amount" of a compound is a quantity sufficient to achieve a desired therapeutic and/or prophylactic effect, for example, an amount which results in the prevention of or a decrease in the symptoms associated with a disease that is being treated, e.g., PD. The amount of compound administered to the subject will depend on the type and severity of the disease and on the characteristics of the individual, such as general health, age, sex, body weight and tolerance to drugs. It will also depend on the degree, severity and type of disease.

As used herein, the term "medical condition" includes, but is not limited to, any condition or disease manifested as one or more physical and/or psychological symptoms for which treatment is desirable, and includes previously and newly identified diseases and other disorders.

As used herein, the term "subject" means that preferably the subject is a mammal, such as a human, but can also be an animal, e.g., domestic animals (e.g., dogs, cats and the like), farm animals (e.g., cows, sheep, pigs, horses and the like) and laboratory animals (e.g., monkey, rats, mice, guinea pigs and the like).

As used herein, the administration of an agent or drug to a subject includes self-administration and the administration by another. It is also to be appreciated that the various modes of treatment or prevention of medical conditions as described are intended to mean "substantial", which includes total but also less than total treatment or prevention, and wherein some biologically or medically relevant result is achieved.

As used herein, the term "excretion behavior" includes, but is not limited to, excretion amount of a metabolite, excretion rate of a metabolite, and change in the amount and rate with the lapse of time.

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description and the claims. In the specification and the appended claims, the singular forms include plural referents unless the context clearly dictates otherwise. Unless defined otherwise, all technical and scientific terms used herein have the same meaning as commonly understood by one of ordinary skill in the art to

which this invention belongs. All references cited herein are incorporated herein by reference in their entirety and for all purposes to the same extent as if each individual publication, patent, or patent application was specifically and individually indicated to be incorporated by reference in its entirety for all purposes.

II. General

L-3,4-dihydroxyphenylalanine (hereinafter, "LD"; a.k.a., (-)-L-a-amino-b-(3,4-dihydroxybenzene) propanoic acid; L-dopa; Levodopa; Dopar®; and Larodopar®), is a naturally occurring amino acid present in both plants and animals. LD is converted into the biologically active amine, dopamine, by DDC enzyme in the animal and human body as shown below. Metabolism of LD by DDC occurs in both peripheral tissue and in the central nervous system. In the mammalian brain, the decarboxylation product dopamine occurs in high amounts in the basal ganglia.

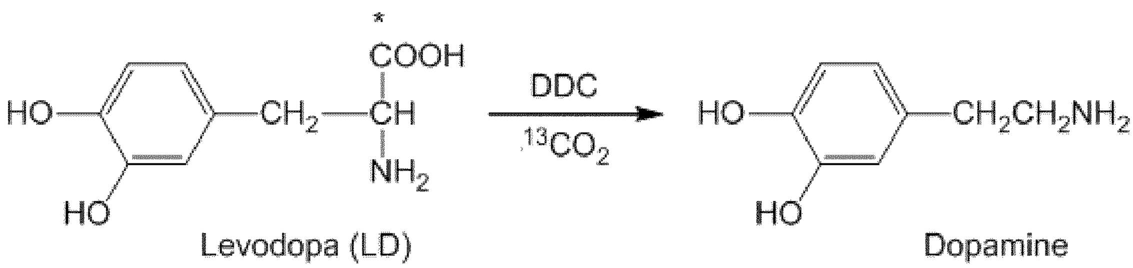

Levodopa (LD) Dopamine

The derangement of dopamine production in the mammalian brain in conditions such as PD can lead to profound central nervous system disturbance. PD is a progressive neurodegenerative disorder that affects 1% of the population over age 65. Currently, about 1 million patients in the U.S. have PD. About 50,000 Americans are diagnosed with PD yearly according to the National Institute of Neurological Disorders and Stroke, which estimates that the total cost of health care for PD patients will exceed $5.6 billion this year.

The symptoms of PD result largely from the loss of dopamine-producing cells in the substantia nigra region of the mammalian brain (see Figure 1). Approximately 50-60% of the normal supply of dopamine has to be depleted before symptoms emerge. At first blush, neurotransmitter replacement therapy using dopamine would seem a logical approach to the management of PD symptoms, however, dopamine does not cross the BBB.

In contrast to dopamine, when LD is taken orally, some of the drug crosses the BBB into the central nervous system. Once LD crosses the BBB, it is enzymatically converted to dopamine (See Figure 2). The resulting increase in brain dopamine concentrations improves nerve conduction and alleviates the movement disorders in PD. Indeed, LD has a strong therapeutic effect in patients with PD. The use of LD as a means of neurotransmitter replacement therapy in PD is now a standard clinical regimen. Although a number of therapies have been developed in an attempt to improve PD management, e.g., dopaminergic agonists and inhibitors of COMT and MAO-B, LD remains the most effective treatment for the symptomatic control of PD.

LD is also decarboxylated systemically in liver, kidney, the gastrointestinal tract and endothelial cells of capillary walls (see Figure 2). This peripheral decarboxylation is responsible for significant side effects in subjects that include nausea, vomiting, cardiac arrhythmias and hypotension. In addition, any LD that is converted to dopamine systemically cannot enter the brain, resulting in diminished central nervous system dopamine level.

Carbidopa (hereinafter, "CD"; (-)-L-$\alpha$-hydrazino-$\alpha$-methyl-b-(3,4-dihydroxybenzene) propanoic acid monohydrate) is an inhibitor of aromatic amino acid decarboxylation that is useful to inhibit peripheral LD decarboxylation by DDC. Unlike LD, CD does not cross the blood-brain barrier. CD is routinely administered as a second drug with LD to inhibit peripheral decarboxylation in the treatment of PD. That is, CD prevents the breakdown of LD before it crosses into the brain.

Standard release combination formulation of CD/LD (Sinemet®) is available in 10mg/100mg, 25mg/100mg and 25mg/250mg and extended release CD/LD (Sinemet CR) in 25mg/100mg and 50mg/200mg tablets. Combination therapy of LD with CD results in less systemic LD breakdown and consequently more LD enters the brain to be converted to dopamine. The addition of CD to the PD therapeutic regimen allows administration of lower total doses of LD to a subject in need thereof. In turn, this reduces the risk of side effects from LD such as nausea and vomiting.

While LD therapy is effective in removing almost all signs and symptoms of the PD within the first three to four years; later, PD patients develop periods of time when medication does not work resulting in an "off" state. During "off spells PD patients revert to the symptoms/signs that characterize untreated PD. This problem is magnified by the fact that late-stage PD patients tend to have an "all or none" response to LD replacement therapy; that is, medication response tends to fluctuate only between good response ("on" state) and no response at all ("off" state). Consequently, in late-stage disease, when a patient is "off her symptoms/signs tend to be extremely severe. For late-stage patients with "on/off

a critical amount of LD in brain (threshold level) is needed to maintain central dopamine activity at a level that results in an "on" state. If brain LD levels drop below this threshold level severe "off" time results. In addition, too much LD resulting in very high brain dopamine levels can lead to dyskinesia, hallucinations and other neurobehavioral disorders. Dyskinesia represents uncontrollable writhing movements that can become more troublesome than the PD signs themselves. This need for ideal control of LD levels is depicted in the picture below (Figure 3).

The amount of LD entering a subject's brain is critical to optimal control of LD therapeutic levels and the consequent clinical benefit of LD. Effective administration of CD to inhibit peripheral decarboxylation of LD is an important factor affecting the amount of LD entering the brain of a subject. For example, where less CD is available or its inhibitory action compromised, peripheral metabolism of LD by DDC is greater and thus less LD is available for DDC-mediated enzymatic conversion in the CNS.

Determination of optimal CD dosage for refinement of LD therapeutic delivery is confounded by individual subject variability in CD absorption and/or responsiveness. Studies employing stable isotope-labeled LD (Durso et al., J. Clin. Pharmacol., 40: 854-860, 2000) showed that absorption of CD is variable among human subjects with significant consequence to the degree of peripheral decarboxylation inhibition among subjects as well as the subsequent level of dopamine replacement in brain (See Figure 4). Subjects can be classified as "good/rapid" CD absorbers (closed circles) and "poor/slow" CD absorbers (open circles). The level of DDC inhibition of individual subjects to the same dose of CD also varies. A priori there is no way of knowing whether a subject is "CD sensitive" and will responded well to CD administration or "CD insensitive" and not show marked inhibition of peripheral DDC with CD administration. Moreover, peripheral DDC activity is not saturated at CD doses used in current clinical practice. (Durso et al., J. Clin. Pharmacol., 40: 854-860, 2000). It is important, therefore, to study the dose dependence of CD on peripheral LD decarboxylation and LD uptake in the brain of individual subjects.

## PREPARATION AND METHODS OF THE INVENTION

### A. Isotope-labeled DDC Enzyme Substrate Preparations of the Invention

The present invention provides preparations for easily determining and assessing the DDC activity {i.e., LD metabolic capacity) in an individual mammalian subject. The preparations are useful for determining the LD metabolite behavior in a subject and easily assessing the LD metabolic capacity and identifying a clinical response and/or medical condition related to DDC activity in the subject. Specifically, the preparations of the invention are useful to determine and assess the LD metabolic capacity in an individual subject by measuring the behavior of a LD metabolite, in particular the excretion behavior of the metabolite (including excretion amount, excretion rate, and change in the amount and rate with the lapse of time), in the subject. The preparation for determining LD metabolic capacity can also be used in combination with at least one DDC enzyme inhibitor (e.g., CD) to determine the dosage of DDC inhibitor required to suppress DDC enzyme in an individual subject as well as to determine the optimal timing for dosing of DDC inhibitor in a subject.

A preparation useful in the methods of the present invention contains an isotopically labeled DDC substrate compound, such as levodopa (LD), tryptophan, phenylalanine, tyrosine, histidine, and 5-hydroxytryptophan, as an active ingredient. In one embodiment, the DDC substrate compound is an LD in which at least one of the carbon or oxygen atoms is labeled with an isotope and the preparation is capable of producing isotope labeled $CO_2$ after administration to a subject. The DDC substrate of the invention can be labeled in at least one position with $^{13}C$; $^{14}C$; and $^{18}O$. In a preferred embodiment, an LD is isotopically labeled with $^{13}C$ such that the preparation is capable of producing $^{13}CO_2$ after administration to a subject.

A preparation of the invention may be formulated with a pharmaceutically acceptable carrier. As used herein, "pharmaceutically acceptable carrier" is intended to include any and all solvents, dispersion media, coatings, antibacterial and antifungal compounds, isotonic and absorption delaying compounds, and the like, compatible with pharmaceutical administration. Suitable carriers are described in the most recent edition of Remington's Pharmaceutical Sciences (Remington: The Science And Practice Of Pharmacy, Lippincott Williams & Wilkins; 21 st Cdr edition (2005), a standard reference text in the field. Supplementary active compounds can also be incorporated into the compositions.

The method for labeling a DDC substrate with an isotope is not limited and may be a conventional method (Sasaki, "5.1 Application of Stable Isotopes in Clinical Diagnosis": Kagaku no Ryoiki (Journal of Japanese Chemistry) 107, "Application of Stable Isotopes in Medicine, Pharmacy, and Biology", pp. 149-163 (1975), Nankodo: Kajiwara, RADIOISOTOPES, 41, 45-48 (1992)). Some isotopically labeled DDC substrate compounds are commercially available, and these commercial products are conveniently usable. For example, $^{13}C$-labeled LD capable of producing $^{13}CO_2$ after administration to a subject is useful in the methods of the invention and is commercially available at Cambridge isotope Laboratories Inc, Andover, MA.

A pharmaceutical composition of the invention is formulated to be compatible with its intended route of administration. Examples of routes of administration include parenteral, e.g., intravenous, intradermal, subcutaneous, oral

(including inhalation), transmucosal, and rectal administration. The preparation of the present invention may be in any form suitable for the purposes of the present invention. Examples of suitable forms include injections, intravenous injections, suppositories, eye drops, nasal solutions, and other parenteral forms; and solutions (including syrups), suspensions, emulsions, tablets

(either uncoated or coated), capsules, pills, powders, subtle granules, granules, and other oral forms. Oral compositions generally include an inert diluent or an edible carrier.

The preparation of the present invention may consist substantially of the isotope-labeled DDC substrate compound, especially LD, as an active ingredient, but may be a composition further containing a pharmaceutically acceptable carrier or additive generally used in this field according to the form of the preparation (dosage form) (composition for determining LD metabolic capacity), as long as the actions and effects of the preparation of the present invention are not impaired. In such a composition, the proportion of the isotope-labeled DDC substrate compound as an active ingredient is not limited and may be from about 0.1 wt.% to about 99 wt.% of the total dry weight of the preparation. The proportion can be suitably adjusted within the above range.

When the preparation of the present invention is formed into tablets, useful carriers include, but are not limited to, e.g., lactose, sucrose, sodium chloride, glucose, urea, starches, calcium carbonate, kaolin, crystalline cellulose, silicic acid, and other excipients; simple syrups, glucose solutions, starch solutions, gelatin solutions, carboxymethyl cellulose, shellac, methyl cellulose, potassium phosphate, polyvinyl pyrrolidone, and other binders; dry starches, sodium alginate, agar powder, laminaran powder, sodium hydrogencarbonate, calcium carbonate, polyoxyethylene sorbitan, fatty acid esters, sodium lauryl sulfate, stearic acid monoglyceride, starches, lactose, and other disintegrators; sucrose, stearic acid, cacao butter, hydrogenated oils, and other disintegration inhibitors; quaternary ammonium bases, sodium lauryl sulfate, and other absorption accelerators; glycerin, starches, and other humectants; starches, lactose, kaolin, bentonite, colloidal silicic acid, and other adsorbents; and purified talc, stearate, boric acid powder, polyethylene glycol, and other lubricants. Further, the tablets may be those with ordinary coatings (such as sugar-coated tablets, gelatin-coated tablets, or film-coated tablets), double-layer tablets, or multi-layer tablets.

When forming the preparation for determining LD metabolic capacity into pills, useful carriers include, for example, glucose, lactose, starches, cacao butter, hydrogenated vegetable oils, kaolin, talc, and other excipients; gum arabic powder, tragacanth powder, gelatin, and other binders; and laminaran, agar, and other disintegrators. Capsules are prepared in a routine manner, by mixing the active ingredient according to the present invention with any of the above carriers and then filling the mixture into hardened gelatin capsules, soft capsules, or the like. Useful carriers for use in suppositories include, for example, polyethylene glycol, cacao butter, higher alcohols, esters of higher alcohols, gelatin, and semisynthetic glyceride.

When the preparation is prepared in the form of an injection, the injection solution, emulsion or suspension is sterilized and preferably isotonic with blood. Useful diluents for preparing the injection include, for example, water, ethyl alcohol, macrogol, propylene glycol, ethoxylated isostearyl alcohol, polyoxylated isostearyl alcohol, and polyoxyethylene sorbitan fatty acid esters. The injection may contain sodium chloride, glucose, or glycerin in an amount sufficient to make an isotonic solution. Also, an ordinary solubilizer, buffer, soothing agent or the like can be added to the injection.

Further, the preparation of the present invention in any of the above forms may contain a pharmaceutically acceptable additive, such as a color, preservative, flavor, odor improver, taste improver, sweetener, or stabilizer. The above carriers and additives may be used either singly or in combination. The amount of the isotope-labeled DDC substrate compound, especially LD (active ingredient) per unit dose of the preparation of the present invention varies depending on the test sample and the kind of active ingredient used, and cannot be generally defined. A preferred amount is, for example, 1 to 300 mg/body per unit dose, preferably 50 to 150 mg/body per unit dose, although it is not limited thereto as long as the above condition is satisfied.

B. Methods of the Invention

A medical condition or clinical response related to DDC enzyme activity (i.e. LD metabolic capacity) in a subject can be easily assessed using the methods of the present invention by administering an isotope-labeled DDC substrate compound to the subject and measuring the excretion behavior (including excretion amount, excretion rate, and change in the amount and rate with the lapse of time) of isotope-labeled $CO_2$ in the expired air. As such, the present invention provides methods to determine the clearance of a isotope-labeled DDC substrate compound in the presence and/or absence of a DDC inhibitor to establish a more effective dosage regimen (formula, dose, number of doses, etc.) of the DDC substrate compound and/or DDC inhibitor for individual subjects based on the DDC enzyme activity in these subjects.

In one embodiment, the invention provides a method for determining LD metabolic capacity, by administering an isotope-labeled LD preparation of the invention to a mammalian subject, and measuring the excretion behavior of an isotope-labeled metabolite excreted from the body. In one embodiment, the isotope-labeled metabolite is excreted from the body as isotope-labeled $CO_2$ (including $^{13}CO_2$, $^{14}CO_2$, and $C^{18}O_2$) in the expired air.

The isotope-labeled metabolite in the test sample can be measured and analyzed by a conventional analysis

technique, such as liquid scintillation counting, mass spectroscopy, infrared spectroscopic analysis, emission spectrochemical analysis, or nuclear magnetic resonance spectral analysis, which is selected depending on whether the isotope used is radioactive or non-radioactive. The $^{13}CO_2$ can be measured by any method known in the art, such as any method that can detect the amount of exhaled $^{13}CO_2$. For example, $^{13}CO_2$ can be measured spectroscopically, such as by infrared spectroscopy. One exemplary device for measuring $^{13}CO_2$ is the UBiT.RTM.-IR300 infrared spectrometer, commercially available from Meretek (Denver, CO, USA.). The subject, having ingested the $^{13}C$-labeled DDC substrate, can exhale into a breath collection bag, which is then attached to the UBiT.RTM.-IR300. The UBiT.RTM.-IR300 measures the ratio of $^{13}CO_2$ to $^{12}CO_2$ in the breath. By comparing the results of the measurement with that of a standard, the amount of exhaled $^{13}CO_2$ can be subsequently calculated. Alternatively, the exhaled $^{13}CO_2$ can be measured with a mass analyzer.

The preparation of the present invention is administered via the oral or parenteral route to a subject and an isotope-labeled metabolite excreted from the body is measured, so that the LD metabolic capacity (existence, nonexistence or degree of LD metabolic disorder (decrease/increase)) in the subject can be determined from the obtained excretion behavior (the behavior of excretion amount and excretion rate with the lapse of time) of the isotope-labeled metabolite. The metabolite excreted from the body varies depending on the kind of the active ingredient used in the preparation. For example, when the preparation comprises isotope-labeled LD as an active ingredient, the final metabolite is isotope-labeled dopamine or isotope-labeled $CO_2$. Preferably, the preparation comprises, as an active ingredient, an isotope-labeled LD that enables the excretion of isotope-labeled $CO_2$ in the expired air as a result of metabolism. Using such a preparation, the LD metabolic capacity (existence, nonexistence, or degree of LD metabolic disorder (decrease/increase)) in a subject can be determined from the excretion behavior (the behavior of excretion amount and excretion rate with the lapse of time) of isotope-labeled $CO_2$, which is obtained by administering the preparation to the subject via the oral or parenteral route and measuring isotope-labeled $CO_2$ excreted in the expired air.

In one embodiment, the invention provides a method for determining LD metabolic capacity in a mammalian subject, by administering an isotope labeled LD preparation of the invention to a subject, measuring the excretion behavior of an isotope-labeled metabolite excreted from the body, and assessing the obtained excretion behavior in the subject. In one embodiment of the method, an isotope-labeled LD preparation is administered to a mammalian subject, the excretion behavior of isotope-labeled $CO_2$ in the expired air is measured, and assessed. In one embodiment of the method, the excretion behavior of isotope-labeled $CO_2$ or a pharmacokinetic parameter obtained therefrom is compared with the corresponding excretion behavior or parameter in a healthy subject with a normal LD metabolic capacity. That is, the LD metabolic capacity in a subject can be assessed by, for example, comparing the excretion behavior (the behavior of excretion amount or excretion rate with the lapse of time) of an isotope-labeled metabolite obtained by the above measurement, with the excretion behavior of the isotope-labeled metabolite in a reference standard, which is measured in the same manner.

Further, in place of, or in addition to, the excretion behavior of an isotope-labeled metabolite, the area under the curve (AUC), excretion rate (in particular, initial excretion rate), maximum excretion concentration ($C_{max}$), slope of the $\delta$ isotope-labeled $CO_2$ (e.g. $\delta^{13}CO_2$) as a function of time or percent dose recovery as a function of time, or a similar parameter (preferably pharmacokinetic parameter) obtained from the excretion behavior (transition curve of the excretion amount) in the subject is compared with the corresponding parameter in reference standard. In one embodiment, the reference standard is the excretion behavior observed in a one or more health subjects.

In one embodiment, LD metabolic capacity is determined by an area under the curve (AUC), which plots the amount of exhaled. Isotope-labeled $CO_2$ (e.g. $\delta^{13}CO_2$) on the y-axis versus the time after the isotope-labeled substrate is ingested. The area under the curve represents the cumulative $\delta$ isotope-labeled $CO_2$ (e.g. $\delta^{13}CO_2$) recovered.

For example, $^{13}CO_2$ is also quantified as $\delta^{13}CO_2$ (a.k.a., DOB) according to the following equation:

$\delta^{13}CO_2$ equals ($\delta^{13}CO_2$ in sample gas minus $\delta^{13}CO_2$ in baseline sample before ingestion of $^{13}C$-labeled DDC substrate) where δ values are calculated (in) by=[($R_{sample}$/$R_{standard}$)-1] X 1000, and "R" is the ratio of the heavy to light isotope ($^{13}CA^{12}C$) in the sample or standard.

$^{13}CO_2$ and $^{12}CO_2$ in exhaled breath samples is measured by IR spectrometry using the UBiT-IR300 (Meretek Diagnostics, Lafayette, CO; 13CO2 urea breath analyzer instruction manual. Lafayette, CO: Meretek Diagnostics; 2002; A1-A2)[12] The amount of $^{13}CO_2$ present in breath samples is expressed as delta over baseline (DOB) that represents a change in the $^{13}CO_2$/$^{12}CO_2$ ratio of breath samples collected before and after $^{13}C$-labeled DDC enzyme substrate ingestion, e.g., $^{13}C$-labeled LD.

$$DOB = \frac{^{13}CO_2}{^{12}CO_2}_{\text{Post dose sample}} - \frac{^{13}CO_2}{^{12}CO_2}_{\text{Pre dose sample}}$$

The amount of $^{13}C$-labeled DDC substrate absorbed and released into the breath as $^{13}CO_2$ is determined for each time point using the equation described by Amarri (Amarri et al., Clin Nutr. 14:149-54 (1995)). These results are expressed as percentage dose recovery (PDR).

The PDR is calculated using the formula:

$$\frac{\frac{(\delta^{13}_{t} - \delta^{13}_{0}) + (\delta^{13}_{t+1} - \delta^{13}_{0})}{2} \times (t_{+1} - t) \times R_{PDB} \times 10^{-3} \times C}{\frac{\text{mg substrate}}{\text{mol. wt.}} \times \frac{P \times n}{100}} \times 100\%$$

wherein $^{13}\delta=[R_s/R_{PDB)}-1]x10^3$
$R_s$ = $^{13}C$:$^{12}C$ in the sample
$R_{PDB}$ = $^{13}C$:$^{12}C$ in PDB (international standard PeeDeeBelemnite) = 0.0112372)
P is the atom % excess
n is the number of labeled carbon positions
$\delta_{t}$, $\delta_{t+1}$, $\delta_0$ are enrichments at times t, $t_{+1}$ and predose respectively
C is the $CO_2$ production rate (C = 300 [mmol/h]*BSA
BSA = $w^{0.5378}$*$h^{0.3963}$*0.024265 (Body Surface Area)
w: Weight (kg)
h: Height (cm)
Cmax is the highest value of DOB from the breath curve following $^{13}C$-labeled DDC substrate. In one embodiment of the method, the $^{13}C$-labeled DDC substrate is $^{13}C$-labeled LD.

As noted above, the invention provides a method for determining the existence, nonexistence, or degree of DDC metabolic disorder (i.e., a medical condition) in a mammalian subject by administering a preparation of the invention to a mammalian subject, measuring the excretion behavior of an isotope-labeled metabolite excreted from the body, and assessing the obtained excretion behavior in the subject. In a preferred embodiment of the method, the isotope-labeled metabolite is excreted from the body as isotope-labeled $CO_2$ in the expired air.

In one embodiment, the invention provides a method for determining the efficacy of a DDC inhibitor to treat a medical condition in a mammalian subject by (a) administering to the subject the DDC inhibitor and an LD in which at least one of the carbon or oxygen atoms is labeled with an isotope, wherein the LD is capable of producing isotope labeled $CO_2$; (b) determining the level of LD metabolism by measuring isotope labeled $CO_2$ produced in the subject; and comparing the level of LD metabolism of the subject to a level of reference standard LD metabolic capacity, wherein a similarity in the level of LD metabolic capacity of the subject compared to the level of LD metabolism of the reference standard indicates that the compound is effective to treat the medical condition in the subject. In one embodiment of the method, the reference standard level of LD metabolic capacity is the level of LD metabolic capacity of the mammalian subject before administration of the DDC inhibitor. In another embodiment of the method, a level of the reference standard LD metabolic capacity is the average of the level of the LD metabolism of a one or more second mammalian subject.

In one embodiment, the invention provides a method for selecting a prophylactic or therapeutic treatment for a subject by (a) determining the phenotype of the subject; (b) assigning the subject to a subject class based on the phenotype of the subject; and (c) selecting a prophylactic or therapeutic treatment based on the subject class, wherein the subject class comprises two or more individuals who display a level of DDC inhibition that is at least about 10 percent lower than a reference standard level of DDC inhibition. The above-mentioned term "phenotype" includes DDC enzyme activity and LD metabolic capacity of the subject. In one embodiment of the method, the subject class comprises two or more individuals who display a level of DDC inhibition that is at least about 10% higher than a reference standard level of DDC inhibition. In one embodiment of the method, the subject class comprises two or more individuals who display a level of DDC inhibition within at least about 10 percent of a reference standard level of DDC inhibition.

In one embodiment, the invention provides a method for selecting a prophylactic or therapeutic treatment for

a subject by (a) administering to a subject the DDC inhibitor CD and an LD in which at least one of the carbon or oxygen atoms is labeled with an isotope, wherein the LD is capable of producing isotope labeled $CO_2$; (b) determining the level of LD metabolic inhibition by CD, by measuring isotope labeled $CO_2$ produced in the subject; and (c) selecting a prophylactic or therapeutic treatment, including the timing of a CD administration, based on the obtained level of LD metabolic inhibition by CD in the subject.

The therapeutic treatment selected can be administering a drug, selecting a drug dosage, and selecting the timing of a drug administration.

In one embodiment, the invention provides a method for selecting a prophylactic or therapeutic treatment for a subject, by (a) determining the phenotype of the subject; (b) assigning the subject to a subject class based on the phenotype of the subject; and (c) selecting a prophylactic or therapeutic treatment based on the subject class, wherein the subject class comprises two or more individuals who metabolize LD at a rate at least about 10 percent higher than a reference standard rate of LD metabolism. The above-mentioned term "phenotype" includes DDC enzyme activity and LD metabolic capacity of the subject. In one embodiment of the method, the subject class comprises two or more individuals who metabolize LD at a rate at least about 10 percent lower than a reference standard rate of LD metabolism. In one embodiment of the method, the subject class comprises two or more individuals who metabolize LD at a rate within at least about 10 percent of a reference standard rate of LD metabolism. The therapeutic treatment selected can be administering a drug, selecting a drug dosage, and selecting the timing of a drug administration.

In one embodiment, the invention provides a method for evaluating LD metabolic capacity, by administering a $^{13}C$-labeled DDC substrate compound to a mammalian subject; measuring $^{13}CO_2$ exhaled by the subject; and determining LD metabolic capacity from the measured $^{13}CO_2$. In one embodiment of the method, the $^{13}C$ -labeled DDC substrate compound is a $^{13}C$-labeled LD. In one embodiment of the method, the $^{13}C$-labeled substrate compound is administered non-invasively. In one embodiment of the method the $^{13}C$-labeled DDC substrate compound is administered intravenously or by oral route. In one embodiment of the method, the exhaled $^{13}CO_2$ is measured spectroscopically. In one embodiment of the method, the exhaled $^{13}CO_2$ is measured by infrared spectroscopy. In another embodiment of the method, the exhaled $^{13}CO_2$ is measured with a mass analyzer. In one embodiment of the method, the exhaled $^{13}CO_2$ is measured over at least three time periods to generate a dose response curve, and the LD metabolic activity is determined from the area under the curve. In one embodiment of the method, the exhaled $^{13}CO_2$ is measured over at least two different dosages of the $^{13}C$-labeled DDC substrate compound. In one embodiment of the method, the exhaled $^{13}CO_2$ is measured during at least the following time points: $t_0$, a time prior to ingesting the $^{13}C$-labeled DDC substrate compound; $t_1$, a time after the $^{13}C$-labeled DDC substrate compound has been absorbed in the bloodstream of the subject; and $t_2$, a time during the first elimination phase. In one embodiment of the method, LD metabolic capacity is determined from as the a slope of $\delta^{13}CO_2$ at time points $t_1$ and $t_2$ calculated according to the following equation: slope = $[(\delta^{13}CO_2)_2-(\delta^{13}CO_2)_1]/(t_2-t_1)$- wherein $\delta^{13}CO_2$ is the amount of exhaled $^{13}CO_2$. In another embodiment of the invention, a DDC inhibitor is administered to the subject before administrating a $^{13}C$-labeled DDC substrate compound. In a preferred embodiment of the invention, the DDC inhibitor is carbidopa.

The method of the present invention can be non-invasive, only requiring that the subject perform a breath test. The present test does not require a highly trained technician to perform the test. The test can be performed at a general practitioners office, where the analytical instrument (such as, e.g., a UBiT.RTM.-IR300) is installed. Alternatively, the test can be performed at a user's home where the home user can send breath collection bags to a reference lab for analysis.

C. Kits of the Invention

Another embodiment of the invention provides a kit for determining LD metabolic capacity. The kit can include $^{13}C$-labeled DDC substrate compound (e.g., $^{13}C$-labeled LD) and instructions provided with the substrate that describe how to determine LD metabolic capacity in a subject. The $^{13}C$-labeled DDC substrate compound can be supplied as a tablet, a powder or granules, a capsule, or a solution. The instructions can describe the method for LD metabolic capacity by using the area under the curve, or by the slope technique, as described above. The kit can include at least three breath collection bags.

In another embodiment, the invention provides a kit comprising a $^{13}C$-labeled DDC substrate compound (e.g., $^{13}C$-labeled LD) and at least one least one DDC inhibitor (e.g., carbidopa); and instructions provided with the substrate that describe how to determine determination of inhibitor dosage and timing of inhibitor dosage in a subject. The kit can include at least six breath collection bags.

The following Examples are presented in order to more fully illustrate the preferred embodiments of the invention. These Examples should in no way be construed as limiting the scope of the invention, as defined by the appended claims.

EXAMPLES

EXAMPLE 1 DETERMINATION OF OPTIMAL CD DOSAGE FOR SUPPRESSION OF PERIPHERAL LD METABOLISM USING THE $^{13}CO_2$ BREATH TEST METHOD OF THE INVENTION

The present studies employed the $^{13}CO_2$ breath test method of the present invention to determine the optimal dose of CD required to optimally suppress peripheral metabolism of $^{13}$C-labeled LD in individual subjects (i.e., Vlt 1 and Vlt 2). Briefly, a pre-breath test sample was collected in normal human subjects after overnight fasting (12 h) in a 1.3 L aluminum bag (Otsuka Pharmaceutical Co., Ltd., Tokyo, Japan). Briefly, normal human subjects ingested varying dosages (e.g., 25 mg-200 mg CD) 1 h prior to administration of 100 mg $^{13}$C-labeled LD on three successive days. Breath samples were collected at specified intervals following administration of $^{13}$C-labeled LD to determine peripheral metabolic decarboxylation of the drug. That is, breath samples were collected at 5 minutes intervals for 40 minutes, at 10 minutes intervals to 90 minutes and 120 minutes. Metabolism of $^{13}$C-labeled LD in the absence of CD administration served as control. The results of these studies are summarized Figures 5-8. There is a dose response to the inhibition of peripheral DDC enzyme activity by the inhibitor CD.

EXAMPLE 2 PREADMINISTRATION OF CD YIELDS SUPERIOR SUPPRESSION OF PERIPHERAL LD DECARBOXYLATION COMPARED TO SIMULTANEOUS CD/LD ADMINISTRATION

The present studies examined the effect of CD preadministration on peripheral LD metabolism in human subjects. Briefly, normal human subjects ingested varying dosages (e.g., 25 mg-200 mg CD) either simultaneously, or up to 2 h prior to administration of $^{13}$C-labeled LD. Breath samples were collected at specified intervals following administration of $^{13}$C-labeled LD to determine peripheral metabolic decarboxylation of the $^{13}$C-labeled LD. Metabolism of $^{13}$C-labeled LD in the absence of CD administration served as control. The results of these studies are summarized below in Table 1 as well as Figures 9-14.

TABLE 1

| CD | $DOB_{20}$ | | $PDR_{40}$ | | $C_{max}$ | |
|---|---|---|---|---|---|---|
| Vlt 1 | 1h prior | simultaneous | 1h prior | simultaneous | 1h prior | simultaneous |
| 0 mg | 45.6 | 45.6 | 29.8 | 29.8 | 58.8 | 58.8 |
| 25 mg | 25.8 (43) | 36.6 (22) | 12.7 (57) | 18.7 (37) | 26.9 (54) | 47.2 (20) |
| 50 mg | 6.8 (85) | 25.5 (44) | 3.6 (88) | 11.7 (61) | 6.8 (88) | 25.5 (57) |
| 100 mg | 10.6 (77) | 16.5 (64) | 5.3 (82) | 9.1 (69) | 10.6 (82) | 17.5 (70) |
| Vlt 2 | 1h prior | simultaneous | 1h prior | simultaneous | 1h prior | simultaneous |
| 0 mg | 36.7 | 36.7 | 18.8 | 18.8 | 36.7 | 36.7 |
| 50 mg | 12.6 (66) | 15.4 (58) | 8.3 (56) | 11.0 (41) | 19.1 (48) | 25 (32) |
| 100 me | 13.5 (63) | 26.7 (27) | 7.8 (59) | 14.1 (25) | 13.5 (63) | 26.7 (27) |
| 200 mg | 8.8 (76) | 20.5 (44) | 6.7 (70) | 12.0 (36) | 9.2 (75) | 20.6 (44) |
| $DOB_{20}$: DOB value at 20 minutes after administration of $^{13}$C-labeled LD<br>$PDR_{40}$: PDR value at 40 minutes after administration of $^{13}$C-labeled LD | | | | | | |

In Table 1, figures in parenthesis are percent inhibition of DDC by CD. The delta over baseline of $^{13}CO_2$ is designated as DOB. The percentage of LD dose recovered as $^{13}CO_2$ is designated as PDR. The peak breath concentration of $^{13}CO_2$ is designated as $C_{max}$.

As shown in Table 1 and Figure 9 through Figure 14, the control level of peripheral metabolism of $^{13}$C-labeled LD in the normal human subjects was significantly inhibited in these individuals after administration of CD at all concentrations of CD tested. The inhibition of the peripheral metabolism of $^{13}$C-labeled LD was also greater when CD was administered prior to $^{13}$C-labeled LD administration (either 1 h or 2 h) compared to the level of LD metabolism observed when CD and $^{13}$C-labeled LD were administered simultaneously (Figure 6, panel E and panel F). Specifically, the simultaneous ingestion of CD/LD with either the 25 mg or 50 mg dose of CD and 100 mg of LD did not completely suppress the peripheral decarboxylation of DCC. If CD was administered 1 h prior to LD dose, however, there was maximal suppression of DCC observed in human subjects (i.e., Vlt 1 and Vlt 2). There was no significant difference in

the level of peripheral metabolism of $^{13}C$-labeled LD observed when CD was administered 1 h prior to LD administration versus administration of CD 2 h prior to LD administration.

EXAMPLE 3 BREATH TEST PROCEDURE

In one embodiment of the breath test procedure of the invention, $^{13}C$-labeleld LD (100 mg) is ingested by a subject after overnight fasting (8-12 h), over a time period of approximately 10-15 seconds. Breath samples are collected at 5 min time points up to 40 min and at 10 min intervals to 90 min and at 120 min after ingestion of $^{13}C$-labeled LD. The breath samples are collected by having the subject momentarily holding their breath for 3 seconds prior to exhaling into a sample collection bag. The breath samples are analyzed on a UBiT IR-300 spectrophotometer (Meretek, Denver, Colo.) to determine the $^{13}CO_2/^{12}CO_2$ ratio in expired breath, or sent to a reference lab. Optionally, varying doses (10-400 mg) of CD are orally administered to the subject (10 min-6 h) prior to administration of the $^{13}C$-labeleld LD.

FURTHER EMBODIMENTS include the following:

1. A preparation for determining levodopa metabolic capacity, comprising as an active ingredient a levodopa in which at least one of the carbon or oxygen atoms is labeled with an isotope, wherein the preparation is capable of producing isotope-labeled $CO_2$ after administration to a mammalian subject.

2. The preparation according to above paragraph 1, wherein the isotope is at least one isotope selected from the group consisting of: $^{13}C$; $^{14}C$; and $^{18}O$.

3. A method for determining levodopa metabolic capacity in a mammalian subject, comprising the steps of administering a preparation according to above paragraph 1 to a mammalian subject, and measuring the excretion behavior of an isotope-labeled metabolite excreted from the body.

4. The method according to above paragraph 3, wherein the isotope-labeled metabolite is excreted from the body as isotope-labeled $CO_2$ in the expired air.

5. The method according to above paragraph 3, further comprising the step of assessing the obtained excretion behavior in the subject.

6. The method according to above paragraph 3, further comprising the step of comparing the obtained excretion behavior in the subject or a pharmacokinetic parameter obtained therefrom with the corresponding excretion behavior or parameter in a healthy subject with a normal levodopa metabolic capacity.

7. A method for determining the existence, nonexistence, or degree of levodopa metabolic disorder in a mammalian subject, comprising the steps of administering a preparation of above paragraph 1 to the subject, and measuring the excretion behavior of an isotope labeled metabolite excreted from the body.

8. The method according to above paragraph 7, further comprising the step of assessing the obtained excretion behavior in the subject

9. A method for determining the efficacy of a dopamine decarboxylase inhibitor to treat a medical condition in a first mammalian subject, the method comprising:

(a) administering to the first subject the dopamine decarboxylase inhibitor and a levodopa in which at least one of the carbon or oxygen atoms is labeled with an isotope, wherein the levodopa is capable of producing isotope labeled $CO_2$;
(b) determining the level of levodopa metabolic capacity by measuring isotope labeled $CO_2$ produced in the first subject; and
(c) comparing the level of levodopa metabolic capacity of the first subject to level of a reference standard levodopa metabolic capacity, wherein a similarity in the level of levodopa metabolic capacity of the first subject compared to level of a reference standard levodopa metabolic capacity indicates that the dopamine decarboxylase inhibitor is effective to treat the medical condition in the first mammalian subject.

10. The method according to above paragraph 9, wherein the level of the reference standard levodopa metabolic capacity is the level of levodopa metabolic capacity of the first subject before administration of the dopamine decarboxylase inhibitor.

11. The method according to above paragraph 9, wherein the level of the reference standard levodopa metabolic capacity is the average of the level of the levodopa metabolic capasity of a one or more second mammalian subject.

12. A method for selecting a prophylactic or therapeutic treatment for a subject, comprising:

(a) determining the levodopa metabolic capacity or dopamine decarboxylase activity of the subject;
(b) assigning the subject to a subject class based on the levodopa metabolic capacity or dopamine decarboxylase activity of the subject; and
(c) selecting a prophylactic or therapeutic treatment based on the subject class, wherein the subject class comprises two or more individuals who display a level of dopamine decarboxylase inhibition that is at least about 10 percent lower than a reference standard level of dopamine decarboxylase inhibition.

13. The method, according to above paragraph 12, which comprising the following step (c’) in . place of (c):

(c’) selecting a prophylactic or therapeutic treatment based on the subject class, wherein the subject class comprises two or more individuals who display a level of dopamine decarboxylase inhibition that is at least about 10% higher than a reference standard level of dopamine decarboxylase inhibition.

14. The method according to above paragraph 12, which comprising the following step (c") in place of (c):

(c") selecting a prophylactic or therapeutic treatment based on the subject class, wherein the subject class comprises two or more individuals who display a level of dopamine decarboxylase inhibition within at least about 10 percent of a reference standard level of dopamine decarboxylase inhibition.

15. The method according to claim 12, wherein the treatment is selected from administering a drug, selecting a drug to be administered, selecting a drug dosage, and selecting the timing of a drug administration.

16. A method for selecting a prophylactic or therapeutic treatment for a subject, comprising:

(a) determining the levodopa metabolic capacity or dopamine decarboxylase activity of the subject;
(b) assigning the subject to a subject class based on the levodopa metabolic capacity or dopamine decarboxylase activity of the subject; and
(c) selecting a prophylactic or therapeutic treatment based on the subject class, wherein the subject class comprises two or more individuals who metabolize levodopa at a rate at least about 10 percent higher than a reference standard rate of levodopa metabolism.

17. The method according to above paragraph 16, which comprising the following step (c’) in place of (c):

(c’) selecting a prophylactic or therapeutic treatment based on the subject class, wherein the subject class comprises two or more individuals who metabolize levodopa at a rate at least about 10 percent lower than a reference standard rate of levodopa metabolism.

18. The method according to claim 16, which comprising the following step (c") in place of (c):

(c") selecting a prophylactic or therapeutic treatment based on the subject class, wherein the subject class comprises two or more individuals who metabolize levodopa at a rate within at least about 10 percent of a reference standard rate of levodopa metabolism.

19. The method according to above paragraph 16, wherein the treatment is selected from administering a drug, selecting a drug to be administered, selecting a drug dosage, and selecting the timing of a drug administration.

20. A method for evaluating levodopa metabolic capacity, comprising the steps of: administering a $^{13}C$-labeled dopamine decarboxylase substrate to a mammalian subject; measuring $^{13}CO_2$ exhaled by the subject; and determining levodopa metabolic capacity from the measured $^{13}CO_2$.

21. The method according to above paragraph 20, wherein the $^{13}C$-labeled dopamine decarboxylase substrate is a $^{13}C$-labeled levodopa.

22. The method according to above paragraph 20, wherein the $^{13}C$-labeled dopamine decarboxylase substrate is administered non-invasively.

23. The method according to above paragraph 20, wherein the $^{13}C$-labeled dopamine decarboxylase substrate is administered intravenously or orally.

24. The method according to above paragraph 20, wherein the exhaled $^{13}CO_2$ is measured spectroscopically or with a mass analyzer.

25. The method according to above paragraph 24, wherein the exhaled $^{13}CO_2$ is measured by infrared spectroscopy.

26. The method according to above paragraph 20, wherein the exhaled $^{13}CO_2$ is measured over at least three time periods to generate a dose response curve, and the levodopa metabolic capacity is determined from the area under the curve.

27. The method according to above paragraph 26, wherein the exhaled $^{13}CO_2$ is measured over at least two different dosages of the $^{13}C$-labeled dopamine decarboxylase substrate.

28. The method according to above paragraph 20, wherein the exhaled $^{13}CO_2$ is measured during at least the following time points: to, a time prior to ingesting the $^{13}C$-labeled dopamine decarboxylase substrate; $t_1$, a time after the $^{13}C$-labeled dopamine decarboxylase substrate has been absorbed in the bloodstream of the subject; and $t_2$, a time during the first elimination phase.

29. The method according to above paragraph 28, wherein the levodopa metabolic capacity is determined from as the a slope of $\delta^{13}CO_2$ at time points $t_1$ and $t_2$ calculated according to the following equation: slope = $[(\delta^{13}CO_2)_2-(\delta^{13}CO_2)_1]/(t_2-t_1)$- wherein $\delta^{13}CO_2$ is the amount of exhaled $^{13}CO_2$.

30. The method according to above paragraph 20, a levodopa inhibitor is administered to the subject before administrating a $^{13}C$-labeled dopamine decarboxylase substrate.

31. A kit comprising: a $^{13}C$-labeled dopamine decarboxylase substrate; and instructions provided with the substrate that describe how to determine $^{13}C$-labeled levodopa metabolic capacity in a subject.

32. The kit according to above paragraph 31, wherein the $^{13}C$-labeled dopamine decarboxylase substrate is $^{13}C$-labeled levodopa.

33. The kit according to above paragraph 31, further comprising at least three breath collection bags.

34. A kit comprising: a $^{13}C$-labeled dopamine decarboxylase substrate and at least one least one dopamine decarboxylase inhibitor; and instructions provided with the substrate that describe how to determine determination of the inhibitor dosage and timing of the inhibitor administration in a subject.

35. The kit according to above paragraph 34, wherein the $^{13}C$-labeled dopamine decarboxylase substrate is $^{13}C$-labeled levodopa and the inhibitor is carbidopa.

36. The kit according to above paragraph 34, further comprising at least six breath collection bags.

37. A use of a levodopa in which at least one of the carbon or oxygen atoms is labeled with an isotope, for producing a preparation for determining levodopa metabolic capacity of a mammalian subject, wherein the preparation is capable of producing isotope-labeled $CO_2$ after administration to the subject.

38. The use according to above paragraph 37, wherein the isotope is at least one isotope selected from the group consisting of: $^{13}C$; $^{14}C$; and $^{18}O$.

39. A use of a levodopa in which at least one of the carbon or oxygen atoms is labeled with an isotope, for producing a preparation for determining the dosage of dopamine decarboxylase inhibitor or timing of the inhibitor administration in a mammalian subject, wherein the preparation is capable of producing isotope-labeled $CO_2$ after administration to the subject.

40. The use according to above paragraph 39, wherein the isotope is at least one isotope selected from the group consisting of: $^{13}C$; $^{14}C$; and $^{18}O$.

EQUIVALENTS

The present invention is not to be limited in terms of the particular embodiments described in this application, which are intended as single illustrations of individual aspects of the invention. Many modifications and variations of this invention can be made without departing from its spirit and scope. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled.

## Claims

**1.** A method for determining the existence, nonexistence, or degree of levodopa metabolic disorder in a mammalian subject, comprising the step of
measuring the excretion behavior of an isotope labeled metabolite excreted by the subject after administering to the subject a preparation comprising levodopa as an active ingredient in which at least one of the carbon or oxygen atoms is labeled with an isotope, wherein the preparation is capable of producing isotope-labeled $CO_2$ after administration to a mammalian subject; and optionally
the method further comprises the step of assessing the obtained excretion behavior in the subject.

**2.** A method for determining the efficacy of a dopamine decarboxylase inhibitor to treat a medical condition in a first mammalian subject, the method comprising:

determining the level of levodopa metabolic capacity by measuring isotope labeled $CO_2$ produced in the first subject after administering to the first subject the dopamine decarboxylase inhibitor and a levodopa in which at least one of the carbon or oxygen atoms is labeled with an isotope, wherein the levodopa is capable of producing isotope labeled $CO_2$; and
comparing the level of levodopa metabolic capacity of the first subject to level of a reference standard levodopa metabolic capacity, wherein a similarity in the level of levodopa metabolic capacity of the first subject compared to level of a reference standard levodopa metabolic capacity indicates that the dopamine decarboxylase inhibitor is effective to treat the medical condition in the first mammalian subject.

**3.** The method according to claim 2, wherein the level of the reference standard levodopa metabolic capacity is the level of levodopa metabolic capacity of the first subject before administration of the dopamine decarboxylase inhibitor; and/or optionally, wherein the level of the reference standard levodopa metabolic capacity is the average of the level of the levodopa metabolic capacity of a one or more second mammalian subject.

**4.** A method for selecting a prophylactic or therapeutic treatment for a subject, comprising:

determining the levodopa metabolic capacity or dopamine decarboxylase activity of the subject;
assigning the subject to a subject class based on the levodopa metabolic capacity or dopamine decarboxylase activity of the subject; and
selecting a prophylactic or therapeutic treatment based on the subject class, wherein the subject class comprises two or more individuals who display a level of dopamine decarboxylase inhibition that differs from a reference standard level of dopamine decarboxylase inhibition.

**5.** A method according to claim 4, wherein the level of dopamine decarboxylase inhibition is at least about 10% higher than a reference standard level of dopamine decarboxylase inhibition; or
wherein the level of dopamine decarboxylase inhibition is within at least about 10 percent of a reference standard level of dopamine decarboxylase inhibition; or wherein the level of dopamine decarboxylase inhibition is at least about 10 percent lower than a reference standard level of dopamine decarboxylase inhibition; and/or optionally wherein the treatment is selected from administering a drug, selecting a drug to be administered, selecting a drug dosage, and selecting the timing of a drug administration.

6. A method for selecting a prophylactic or therapeutic treatment for a subject, comprising:

determining the levodopa metabolic capacity or dopamine decarboxylase activity of the subject;
assigning the subject to a subject class based on the levodopa metabolic capacity or dopamine decarboxylase activity of the subject; and
selecting a prophylactic or therapeutic treatment based on the subject class, wherein the subject class comprises two or more individuals who metabolize levodopa at a rate that differs from a reference standard rate of levodopa metabolism.

7. A method according to claim 6, wherein the rate of levodopa metabolization is at least about 10 percent lower than a reference standard rate of levodopa metabolism, or-wherein the rate of levodopa metabolization is within at least about 10 percent of a reference standard rate of levodopa metabolism.

8. The method according to claim 6, wherein the rate of levodopa metabolization is at least about 10 percent higher than a reference standard rate of levodopa metabolism.

9. The method according to any one of claims 6 to 8, wherein the treatment is selected from administering a drug, selecting a drug to be administered, selecting a drug dosage, and selecting the timing of a drug administration.

10. A kit comprising: a $^{13}C$-labeled dopamine decarboxylase substrate; at least one breath collection bag; and instructions provided with the substrate that describe how to determine $^{13}C$-labeled levodopa metabolic capacity in a subject.

11. The kit according to claim 16, wherein the $^{13}C$-labeled dopamine decarboxylase substrate is $^{13}C$-labeled levodopa; and/or optionally the kit, further comprises at least three breath collection bags.

12. A kit comprising: a $^{13}C$-labeled dopamine decarboxylase substrate; at least one dopamine decarboxylase inhibitor; at least one breath collection bag; and instructions provided with the substrate that describe how to determine determination of the inhibitor dosage and timing of the inhibitor administration in a subject.

13. The kit according to claim 12, wherein the $^{13}C$-labeled dopamine decarboxylase substrate is $^{13}C$-labeled levodopa and the inhibitor is carbidopa; and optionally further comprising at least six breath collection bags.

14. A use of a levodopa in which at least one of the carbon or oxygen atoms is labeled with an isotope, for producing a preparation for determining the dosage of dopamine decarboxylase inhibitor or timing of the inhibitor administration in a mammalian subject, wherein the preparation is capable of producing isotope-labeled $CO_2$ after administration to the subject.

15. The use according to claim 14, wherein the isotope is at least one isotope selected from the group consisting of: $^{13}C$; $^{14}C$; and $^{18}O$.

FIGURE 1

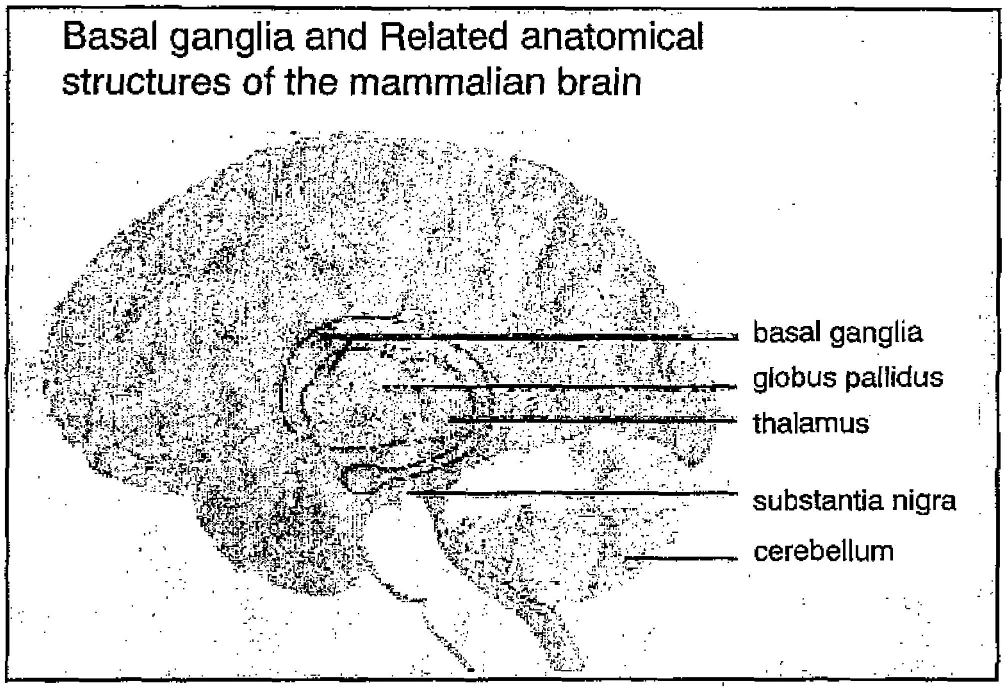


Basal ganglia and Related anatomical structures of the mammalian brain

FIGURE 2

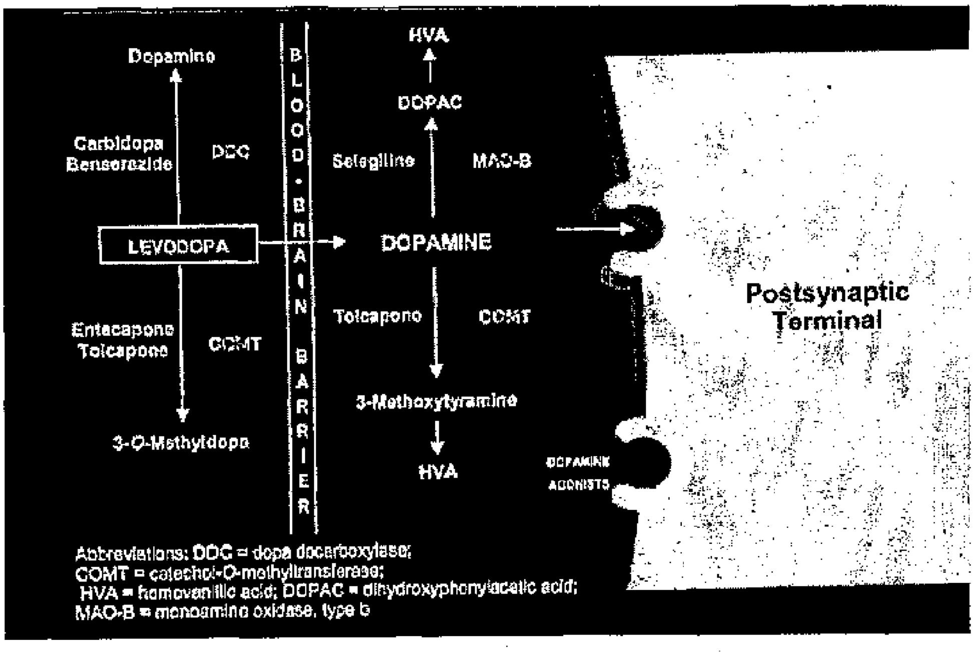
Dopamine
Carbidopa
Benserazide
DDC
LEVODOPA
Entacapone
Tolcapone
COMT
3-O-Methyldopa
BLOOD-BRAIN BARRIER
HVA
DOPAC
Selegiline
MAO-B
DOPAMINE
Tolcapone
COMT
3-Methoxytyramine
HVA
DOPAMINE
AGONISTS
Postsynaptic
Terminal
Abbreviations: DDC = dopa decarboxylase;
COMT = catechol-O-methyltransferase;
HVA = homovanillic acid; DOPAC = dihydroxyphenylacetic acid;
MAO-B = monoamine oxidase, type B

FIGURE 3

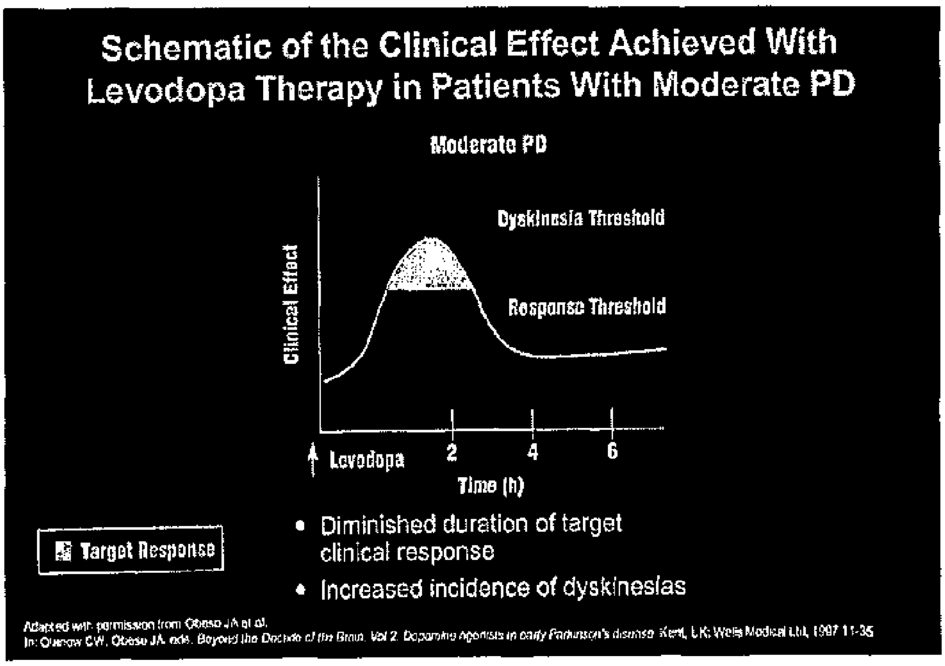
Schematic of the Clinical Effect Achieved With
Levodopa Therapy in Patients With Moderate PD
Moderate PD
Dyskinesia Threshold
Response Threshold
Clinical Effect
Levodopa
2
4
6
Time (h)
Target Response
• Diminished duration of target clinical response
• Increased incidence of dyskinesias
Adapted with permission from Obeso JA et al.

FIGURE 4

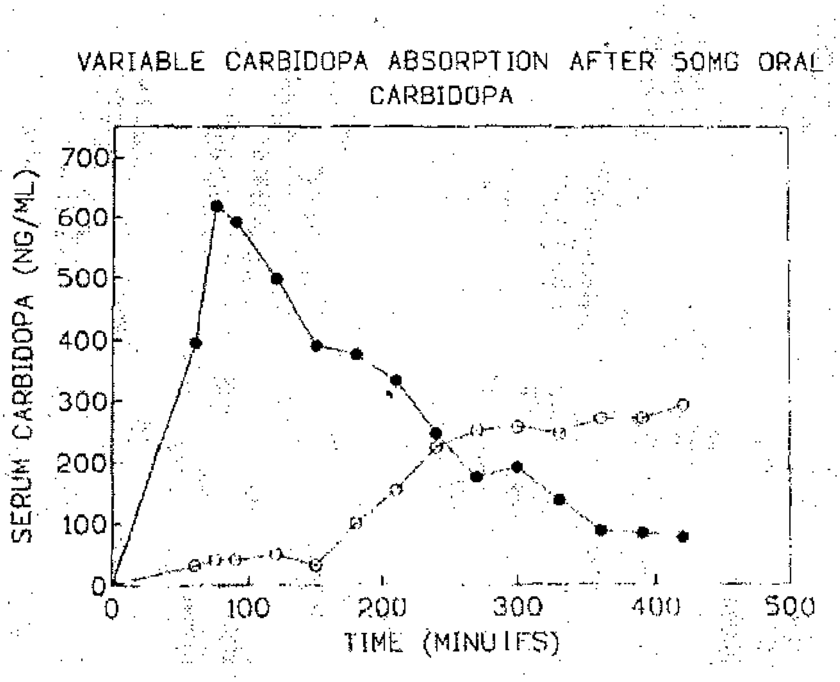
VARIABLE CARBIDOPA ABSORPTION AFTER 50MG ORAL CARBIDOPA
SERUM CARBIDOPA (NG/ML)
700
600
500
400
300
200
100
0
0
100
200
300
400
500
TIME (MINUTES)

FIGURE 5

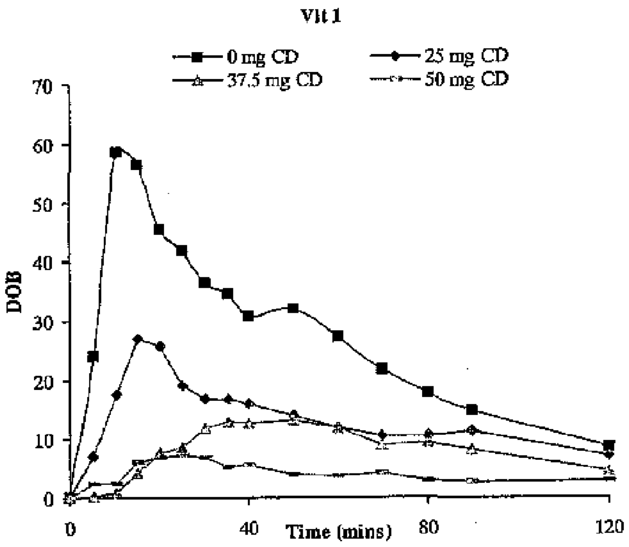


FIGURE 6

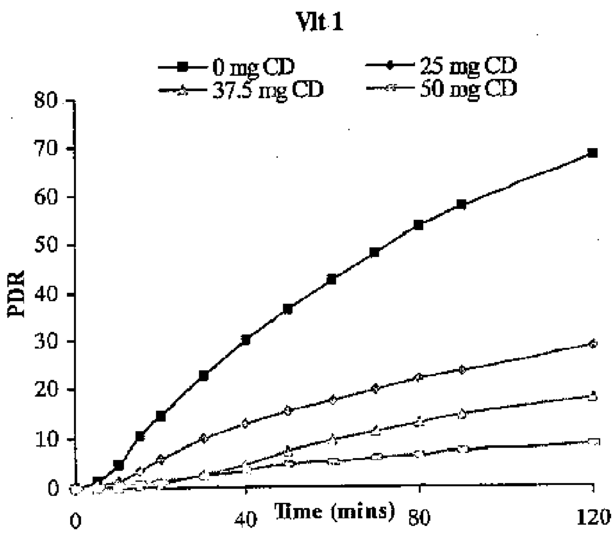

FIGURE 7

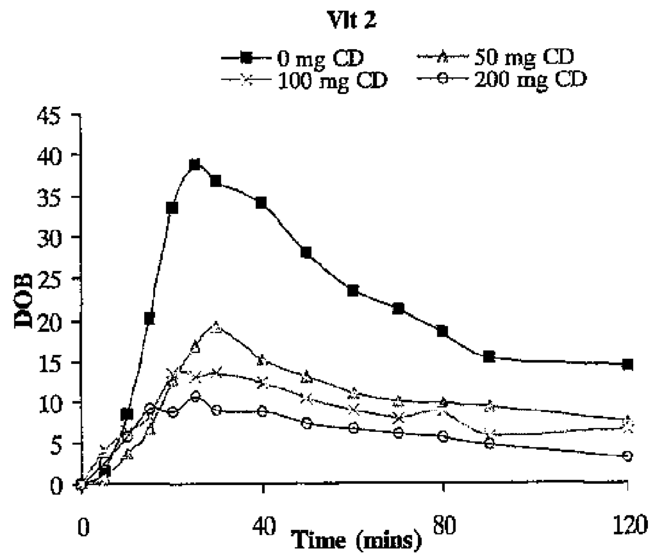


FIGURE 8

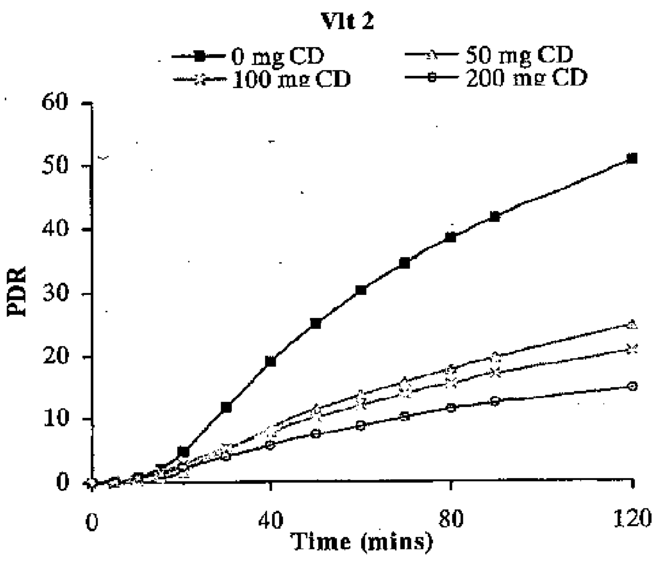

FIGURE 9

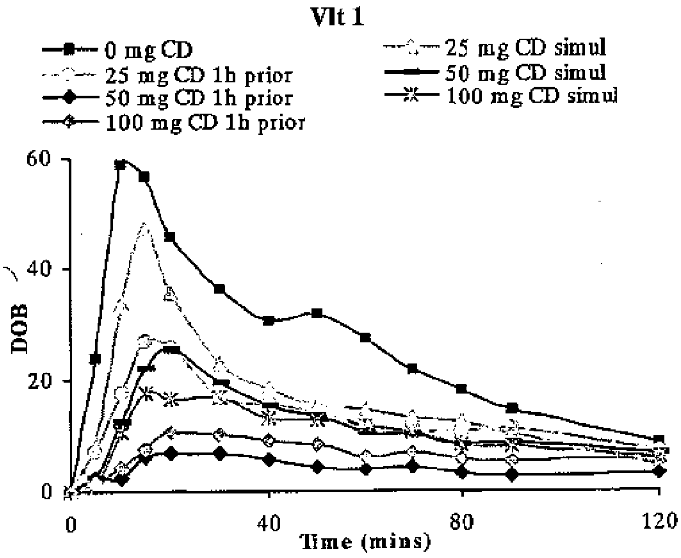


FIGURE 10

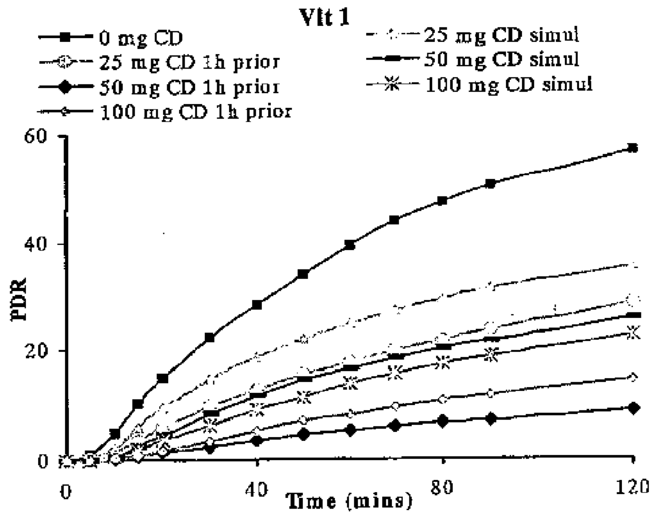

FIGURE 11

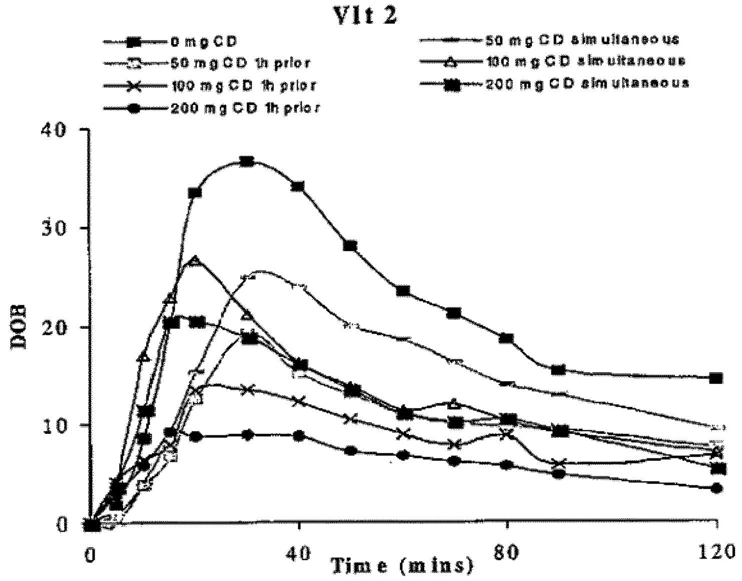


FIGURE 12

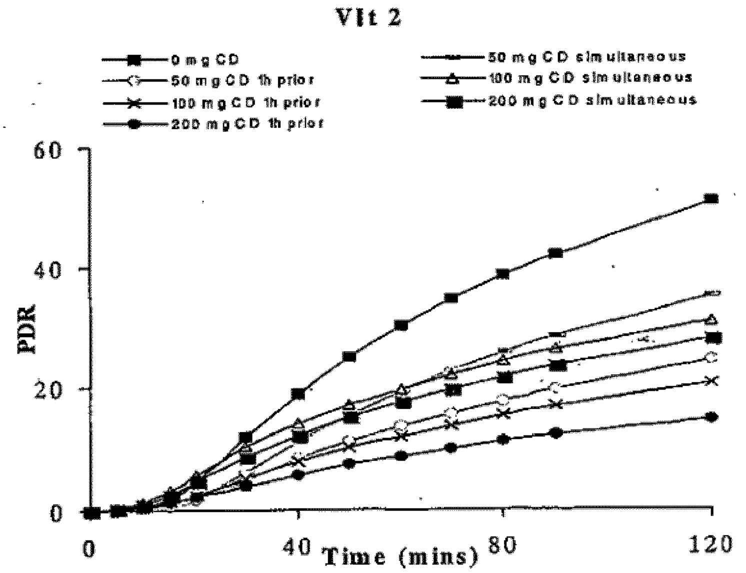

FIGURE 13

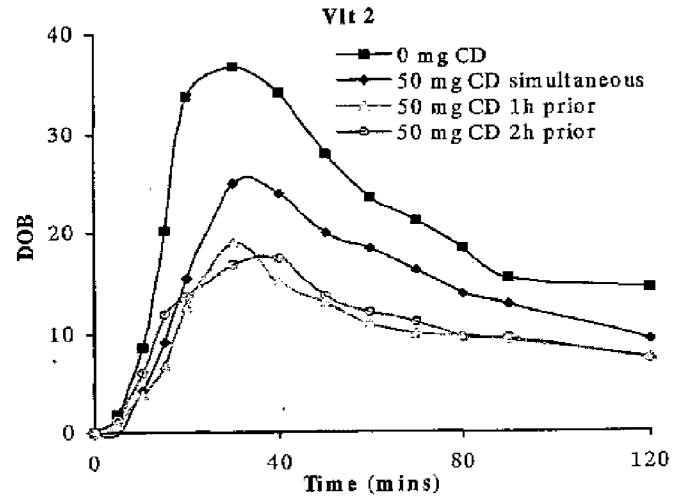


FIGURE 14

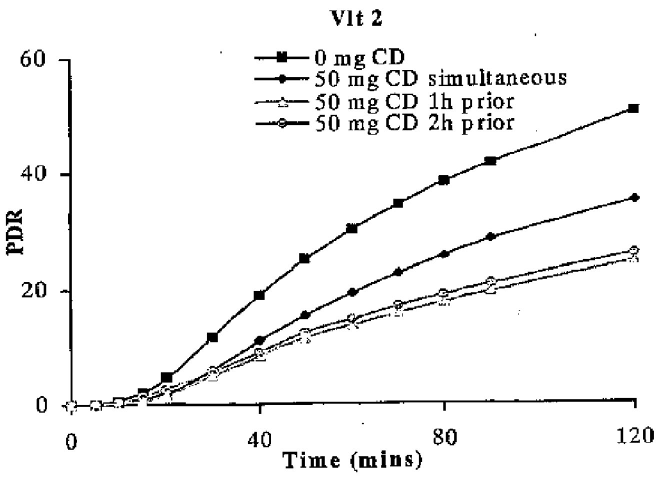

Europäisches Patentamt
European Patent Office
Office européen des brevets

# EUROPEAN SEARCH REPORT

Application Number
EP 14 19 3736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 486 785 A1 (TOKYO GAS CO LTD [JP]) 15 December 2004 (2004-12-15) * par.[0040]; [0048]; [0094]-[0099] * ----- | 1-15 | INV. A61K51/00 A61K31/198 |
| X | DEGRAZIA J A ET AL: "Radiorespirometry studies of the effects of L-dopa and MK486 therapy. Model application of carbon isotopes to the in vivo evaluation of intermediary metabolism during clinical drug trials", PROCEEDINGS OF A SEMINAR ON THE USE OF STABLE ISOTOPES INCLINICAL PHARMACOLOGY, XX, XX, 1 January 1972 (1972-01-01), pages 71-98, XP003003805, * p.74, para.1-p.75, para.2; fig.7-8 * ----- | 1-15 | |

TECHNICAL FIELDS SEARCHED (IPC)
A61K

The present search report has been drawn up for all claims

1

| Place of search | Date of completion of the search | Examiner |
|---|---|---|
| Munich | 4 May 2015 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 14 19 3736

This annex lists the patent family members relating to the patent documents cited in the above-mentioned European search report. The members are as contained in the European Patent Office EDP file on
The European Patent Office is in no way liable for these particulars which are merely given for the purpose of information.

04-05-2015

| Patent document cited in search report | Publication date | Patent family member(s) | Publication date |
|---|---|---|---|
| EP 1486785 A1 | 15-12-2004 | AT 503998 T | 15-04-2011 |
| | | AU 2003211602 A1 | 09-09-2003 |
| | | CA 2474993 A1 | 28-08-2003 |
| | | EP 1486785 A1 | 15-12-2004 |
| | | MX PA04008180 A | 16-05-2005 |
| | | NZ 534462 A | 29-06-2007 |
| | | PT 1486785 E | 12-05-2011 |
| | | US 2005079564 A1 | 14-04-2005 |
| | | WO 03071271 A1 | 28-08-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DELEU et al.** *Eur J Clin Pharmacol.,* 1991, vol. 41, 453-8
- **DURSO et al.** *Clin. Pharmacol.,* 2000, vol. 40, 854-860
- **DURSO et al.** *J. Clin, Pharmacol.,* 2000, vol. 40, 854-860
- Beyond the Decade of the Brain. Dopamine agonists in early Parkinson's disease. Wells Medical Ltd, 1997, vol. 2, 11-35
- **DURSO et al.** *J. Clin. Pharmacol.,* 2000, vol. 40, 854-860
- Remington: The Science And Practice Of Pharmacy. Lippincott Williams & Wilkins, 2005
- **SASAKI.** 5.1 Application of Stable Isotopes in Clinical Diagnosis. *Kagaku no Ryoiki,* 107
- Application of Stable Isotopes in Medicine, Pharmacy, and Biology. Nankodo, 1975, 149-163
- **KAJIWARA.** *RADIOISOTOPES,* 1992, vol. 41, 45-48
- Meretek Diagnostics, Lafayette, CO; CO2 urea breath analyzer instruction manual. Meretek Diagnostics. 2002, A1-A2
- **AMARRI et al.** *Clin Nutr.,* 1995, vol. 14, 149-54